# EUROPÄISCHE PATENTSCHRIFT

(11) **EP 0 510 624 B1**
(45) Veröffentlichungstag und Bekanntmachung des Hinweises auf die Patenterteilung: **05.07.1995**
(21) Anmeldenummer: 92106912.6
(22) Anmeldetag: 23.04.1992
(51) Int. Cl.: A61M 25/01

(54) **Führungskatheter mit vorgebogenem Endbereich**
Guide catheter with pre-bent end region
Cathéter à guidage avec région terminale précourbée

(30) Priorität: 26.04.1991 DE 4113703
(43) Veröffentlichungstag der Anmeldung: 28.10.1992
(73) Patentinhaber: Osypka, Thomas, 79639 Grenzach-Wyhlen (DE)
(72) Erfinder: Osypka, Thomas, 79639 Grenzach-Wyhlen (DE)
(74) Vertreter: Schmitt, Hans, Dipl.-Ing.

(56) Entgegenhaltungen:
- EP-A- 0 226 701
- EP-A- 0 416 734
- DE-A- 3 900 738
- FR-A- 2 639 237

## Beschreibung

Die Erfindung betrifft einen Führungskatheter mit einem inneren Kanal zum Einführen eines Behandlungskatheters oder dergleichen, wobei der dem distalen Austrittsende benachbarte Bereich des Führungskatheters vorgebogen, gekrümmt oder verformbar und der gesamte Führungskatheter zur Positionierung beim Einführen oder in Gebrauchsstellung drehbar ist.

Ein derartiger Führungskatheter ist aus der EP-A-0 416 734 bekannt.

Derartige Führungskatheter dienen in erster Linie dazu, Behandlungskatheter wie zum Beispiel Ballonkatheter zur Erweiterung von Koronararterien des Herzens aufzunehmen und zu führen. Sie müssen daher oft zum Beispiel in der Aorta gedreht und so manipuliert werden, daß sie ohne Schwierigkeiten von der Aorta aus in die Mündung oder Abzweigung einer Koronararterie eingeführt werden können.

Zur Verbesserung der Drehstabilität des Führungskatheters ist es bekannt, dessen Wand durch einen mittels Drahtgeflecht verstärkten Katheterschlauch zu bilden. Andererseits soll jedoch die distale Spitze des Führungskatheters weich sein, um am Zielpunkt oder im Inneren des Zielorganes keine Verletzungen hervorzurufen. Man erreicht dies dadurch, daß der proximale Teil des Führungskatheters durch ein Metallgeflecht verstärkt wird, während der distale Teil diese Verstärkung nicht aufweist.

Die Verbesserung der Drehstabilität durch ein Drahtgeflecht wird dadurch erkauft, daß bei gleichbleibendem Innenlumen der Außendurchmesser eines solchen Führungskatheters größer als ein solcher ohne Drahtgeflecht ist. Dies hat zur Folge, daß die Erweiterung des Gefäßes entsprechend groß gemacht werden muß, wodurch die Komplikationsrate während des Eingriffes steigt und die Heilung verzögert werden kann.

Es besteht deshalb die Aufgabe, einen Führungskatheter der eingangs erwähnten Art zu schaffen, der während seiner Plazierung in dem zu drehenden Bereich die erforderliche Steifigkeit und Drehstabilität hat, trotzdem aber keine vergrößerten Außenabmessungen und Aussteifungen seines Mantels benötigt.

Die überraschende Lösung dieser scheinbar widersprüchlichen Aufgabe besteht bei einem Führungskatheter der eingangs erwähnten Art darin, daß er ein in seinem Inneren vorschiebbares und an seinem distalen Endbereich lösbar festlegbares Drehelement aus torsionsfestem Werkstoff aufweist.

Es wird also eine temporäre drehstabile Verstärkung in den Führungskatheter eingebracht, die am distalen Teil des Katheters insbesondere kraftschlüssig fixierbar ist und dadurch der Spitze des Katheters die gewünschte Drehstabilität verleiht. Nach der Plazierung des Katheters und seines distalen Endes am gewünschten Ort kann dieser Hilfsmechanismus leicht wieder entfernt werden. Somit kann der Führungskatheter ohne Drahtgeflecht mit sehr dünner Wandstärke aufgebaut werden und aus einem einheitlichen zusammenhängenden Schlauchmaterial hergestellt werden, was erheblich zur Kostenreduzierung bei der Produktion beiträgt. Die temporäre Festlegung geschieht dabei so, daß das Drehelement nach allen Seiten, also auch in Rückzugsrichtung festliegt, um die gewünschte Manipulation beim Einführen des Endes des Führungskatheters in eine Gefäßabzweigung auch unter schwierigen räumlichen Bedingungen zu erlauben.

Eine ganz besonders vorteilhafte und zweckmäßige Ausgestaltung der Erfindung von erheblicher Bedeutung für die Einfachheit der Bedienung einerseits und die Effektivität der Drehübertragung im Endbereich des Führungskatheters andererseits kann darin bestehen, daß das Drehelement ein flexibler, aber drehfester Hohlkörper, insbesondere ein Rohr oder Schlauch ist, welcher an seinem distalen Ende zum Verklemmen mit der Innenseite des Führungskatheters aufweitbar oder spreizbar ist. Durch die Spreizung des Endes dieses drehfesten Hohlkörpers kann er gegen die Innenwand des Führungskatheters gedrückt werden und so die gewünschte kraftschlüssige oder gegebenenfalls auch formschlüssige Verbindung herstellen. Durch diese Maßnahme kann eine Drehstabilität von nahezu 1 : 1 erreicht werden, das heißt, die Drehung dieses als Hohlkörper ausgebildeten Drehelementes an seinem proximalen Ende wird praktisch vollständig und ohne Torsionen über die Länge des Führungskatheters an dessen distales Ende übertragen, was die Bedienung durch den Operateur erheblich verbessert. Dies gilt vor allem dann, wenn das Drehelement, insbesondere das Rohr oder der Schlauch, aus Metall besteht, weil ein metallischer Hohlkörper die bei der Plazierung von Führungskathetern auftretenden Drehkräfte nahezu ohne eigene Verformung übertragen kann.

Das distale Ende des Drehelementes kann geschlitzt sein und es kann ein in axialer Richtung des Drehelementes verschiebbares Spreizteil zum Aufweiten des geschlitzten Endes gegen eine Rückstellkraft vorgesehen sein. Durch axiale Verstellung des Spreizteiles in den geschlitzten Bereich des dort hohlen Drehkörpers wird also eine Durchmesservergrößerung erreicht und damit die Verklemmung mit der Innenseite des Führungskatheters auf einfache Weise hergestellt. Die entgegengesetzte Verstellung des Spreizelementes läßt die geschlitzten Enden aufgrund der Rückstellkraft wieder zusammengehen, so daß anschließend das Drehelement wieder problemlos aus dem Führungskatheter herausgezogen werden kann.

Als axial bewegliches Spreizteil kann ein vor dem Spreizen außerhalb der Mündung des Rohres oder Schlauches und seines geschlitzten Bereiches angeordneter, in axialer Richtung gegen das Innere des Rohres oder Schlauches hin sich verjüngender Spreizkörper, insbesondere ein Konus, Kegel oder Keil vorgesehen sein, der mit einem Zugelement verbunden ist, und das Zugelement kann durch das Rohr oder dergleichen bis zu dessen proximalem Ende verlaufen und an dem proximalen Ende austreten. Wird an diesem Zugelement gezogen, gelangt der Spreizkörper in den geschlitzten Bereich und kann diesen aufweiten. Dabei ist das Zugelement gleichzeitig auch so steif, daß es auf eine entgegengesetzt gerichtete Relativbewegung des Spreizkörpers wieder aus dem Drehelement heraus gestattet.

Eine abgewandelte Ausführungsform kann darin bestehen, daß das Spreizteil im Inneren eines sich zur Mündung insbesondere konisch verjüngenden geschlitzten Bereiches des Drehrohres oder dergleichen angeordnet ist und eine mandrinartige Verbindung durch das Rohr bis zu dessen proximalem Ende verläuft und aus diesem mindestens um das Maß vorsteht, um welches das Spreizteil zu der Mündung hin vorschiebbar ist. In diesem Falle erfolgt die Spreizung dadurch, daß das Spreizteil durch Vorschieben des Mandrins in den sich verjüngenden geschlitzten Bereich gelangt und diesen dadurch aufweitet, während zum Lösen des Drehelementes von der Innenwand des Führungskatheters der Mandrin und das Spreizteil wieder zurückziehbar sind.

Eine besonders feinfühlige und präzise Durchführung der Relativbewegung zwischen Spreizteil und Drehelement kann dadurch erreicht werden, daß zur Durchführung der relativen Axialbewegung des Spreizkörpers am proximalen Ende des Rohres oder Schlauches ein Verstellgewinde vorgesehen ist, wobei insbesondere das axial verschiebliche Teil ein Gewinde aufweist und das Rohr oder ein damit verbundenes Lagerteil das dazugehörende Gegengewinde hat. Durch eine Drehbewegung an dem proximalen Ende des Zugelementes oder Mandrins kann also die gewünschte Relativbewegung in axialer Richtung des Führungskatheters durchgeführt werden, wobei eine ganze Umdrehung zu einer Axialverstellung um die Gewindesteigung führt, also ein sehr feinfühliges Arbeiten ermöglicht wird.

Der Spreizteil zum Aufspreizen und Verklemmen des distalen Endes des Rohres im Führungskatheter kann etwa birnenförmig ausgebildet sein, wobei die verdickte Seite das distale Ende des Spreizkörpers bildet und der sich verjüngende Teil zu dem proximalen Ende hin gerichtet ist und das Zugelement zum Einziehen in das geschlitzte Rohrende für dessen Aufspreizung aufweist. Die Birnen- oder Tropfenform hat den Vorteil, daß das Einführen des Drehelementes mit dem vor seiner Mündung befindlichen Spreizteil somit sehr leicht auch durch Biegungen und Kurven des Führungskatheters hindurch erfolgen kann.

Hat die Spitze des Führungskatheters ihre gewünschte Position erreicht, kann die Form- oder Kraftschlüssigkeit zwischen Drehrohr und Katheter dadurch wieder gelöst werden, daß der Spreizteil aus dem Spreizbereich herausbewegt wird, insbesondere der innere Mandrin distal nach vorne geschoben wird, so daß die Schlitzaufweitung aufgrund ihrer Elastizität in ihre Ruhestellung zurückkehren kann. Nunmehr kann das Drehrohr mit dem Mandrin und dem Spreizteil leicht aus dem Führungskatheter herausgezogen werden, um Platz für den eigentlichen Behandlungskatheter zu machen.

Nachstehend ist ein Ausführungsbeispiel der Erfindung anhand der Zeichnung näher beschrieben. Es zeigt in zum Teil schematisierter Darstellung:
- Fig. 1: einen Schnitt durch ein Herz, in welches ein an seinem Ende zweifach vorgekrümmter Führungskatheter eingeführt ist, der mit Hilfe eines in seinem Inneren befindlichen, bis zu dem gekrümmten Bereich vorgeschobenen Drehelement manipulierbar ist,
- Fig. 2: in vergrößertem Maßstab einen Teillängsschnitt durch einen Führungskatheter mit abgewandelter Formgebung seines vorgebogenen Bereiches, bis zu dessen Anfang ein Drehelement vorgeschoben ist, welches aber noch nicht mit der Innenseite des Führungskatheters verklemmt ist,
- Fig. 3: die wesentlichen Teile eines aus einem geschlitzten Rohr und einem Spreizteil mit Mandrin bestehenden Drehelementes vor ihrem Zusammenfügen sowie
- Fig. 4: in vergrößertem Maßstab einen Längsschnitt durch einen Führungskatheter mit in seinem Inneren angeordneten Drehelement in Gebrauchsstellung, in der das distale Ende des Drehelementes aufgespreizt ist, wobei für die Relativbewegung zwischen Drehelement und Spreizteil am proximalen Ende eine Gewindeverbindung vorgesehen ist.

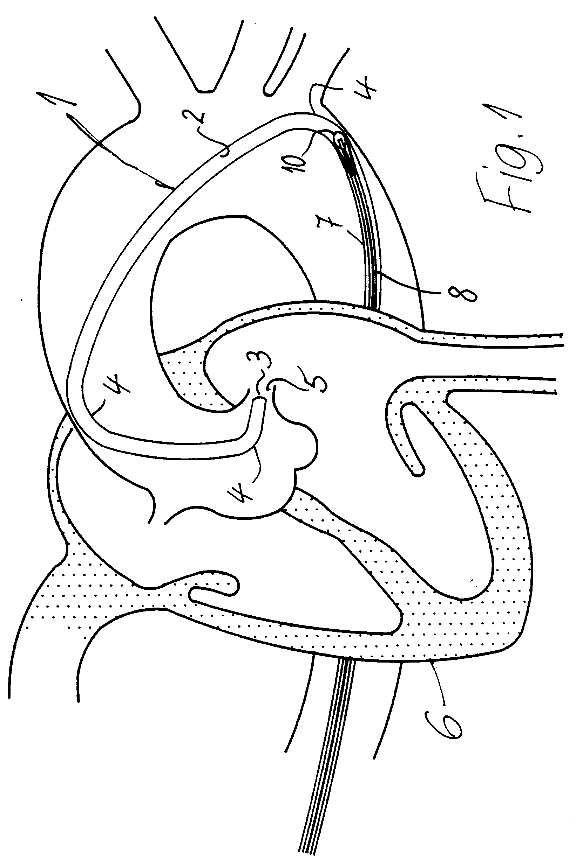

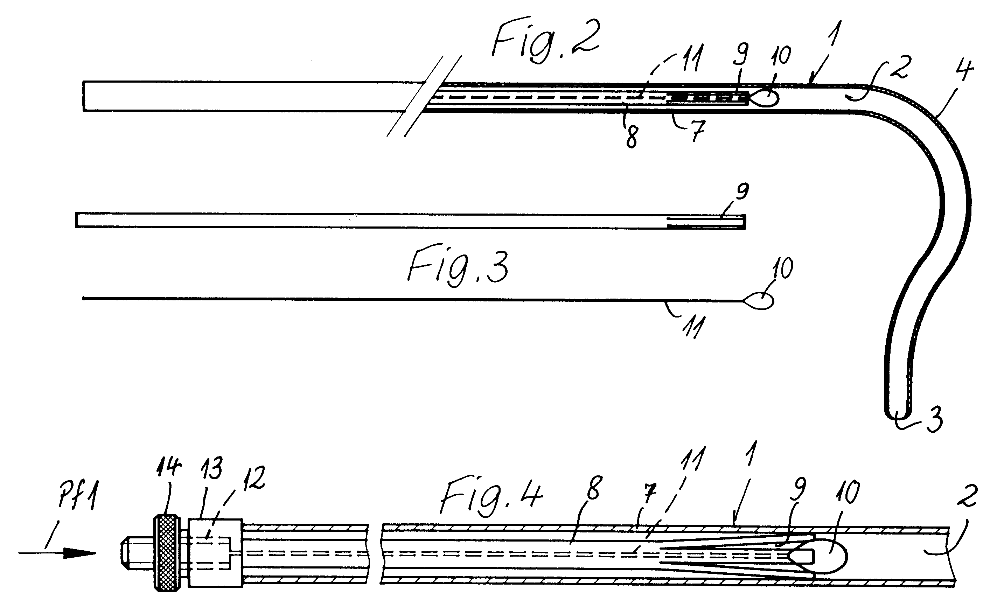

Ein im ganzen mit 1 bezeichneter Führungskatheter hat einen inneren Kanal 2 zum Einführen eines Behandlungskatheters oder dergleichen, wobei der dem distalen Austrittsende 3 benachbarte Bereich des Führungskatheters 1 in unterschiedlicher Weise vorgebogen, gekrümmt oder verformbar sein kann, um eine bestimmte Krümmung vorzuwählen. Fig. 1 zeigt einen insgesamt drei Biegungen 4 aufweisenden vorgebogenen Endbereich, während in Fig.2 ein Führungskatheter mit einer Biegung 4 dargestellt ist. Dieser Führungskatheter 1 ist zu seiner Positionierung beim Einführen und auch in Gebrauchsstellung in noch zu beschreibender Weise drehbar, so daß die Mündung 3 beispielsweise in eine besondere Öffnung 5 am Herzen 6 eingeführt werden kann.

Um die erforderliche Drehkraft nicht über die Wandung 7 des Führungskatheters 1 übertragen zu müssen, was zu einer Torsion des gesamten Führungskatheters 1 über seine Länge führen könnte, hat der Führungskatheter ein in seinem Inneren vorschiebbares und an seinem distalen Endbereich lösbar festlegbares Drehelement 8 aus torsionsfestem Werkstoff. Gem. den Figuren 2 bis 4 ist dieses Drehelement 8 ein flexibler, aber drehfester Hohlkörper, zum Beispiel ein Rohr, welcher an seinem distalen Ende zum Verklemmen mit der Innenseite des Führungskatheters 1 aufweitbar oder spreizbar ist. Zu diesem Zweck ist das distale Ende des Drehelementes 8 mit am Ende freimündenden Schlitzen 9 versehen und weist ein in axialer Richtung des Drehelementes 8 verschiebbares Spreizteil 10 zum Aufweiten des geschlitzten Endes gegen die Rückstellkraft seines Werkstoffes auf.

Dabei könnte das Spreizteil 10 im Inneren eines sich zur Mündung konisch verjüngenden geschlitzten Bereiches des Drehrohres 8 angeordnet sein und durch Vorschieben in diesen geschlitzten Bereich zu der erwähnten Spreizung und Verklemmung mit der Innenseite des Führungskatheters 1 führen.

Im Ausführungsbeispiel ist jedoch vorgesehen, daß als axial bewegliches Spreizteil 10 ein vor dem Spreizen außerhalb der Mündung des Rohres 8 - oder eines Schlauches - und seines geschlitzten Bereiches angeordneter, in Richtung gegen das Innere des Rohres hin sich verjüngender Spreizkörper mit konus- oder kegelförmiger Kontur vorgesehen ist, der mit einem Zugelement 11 verbunden ist, wobei dieses Zugelement 11 durch das Rohr 8 bis zu dessen proximalem Ende verläuft und an diesem austritt. Das Zugelement 11 hat dabei gleichzeitig eine solche Steifigkeit, daß es auch Schubkräfte auf das Spreizteil 10 übertragen kann, um es aus der in Fig.4 dargestellten Klemmposition in Richtung des Pfeiles Pf 1 wieder herausschieben und so die Klemmverbindung lösen zu können. Zweckmäßigerweise ist das Zugelement ein Mandrin.

In Fig. 4 erkennt man , daß zur Durchführung der relativen Axialbewegung des Spreizkörpers 10 am proximalen Ende des Drehelementes 8, also des Rohres oder Schlauches, ein Verstellgewinde 12 vorgesehen ist, wobei das axial verschiebliche Teil, also ein Ansatz an dem Mandrin 11, ein Gewinde aufweist und das Drehelement 8 , also das Rohr oder ein damit verbundenes Lagerteil 13 das dazugehörende Gegengewinde hat. Mit Hilfe eines Drehgriffes 14 kann die Gewindeverbindung 12 dazu ausgenutzt werden, die axiale Relativbewegung zwischen Drehelement 8 und Spreizkörper 10 feinfühlig durchzuführen.

Der Spreizteil 10 ist etwa birnenförmig oder tropfenförmig ausgebildet, wobei die verdickte Seite das distale Ende dieses Spreizkörpers 10 bildet und der sich verjüngende Teil zu dem proximalen Ende hin gerichtet ist und das Zugelement beziehungsweise der Mandrin 11 zum Einziehen in das geschlitzte Rohrende aufweist. Beim Einführen des Drehelementes 8 zusammen mit seinem Spreizkörper 10 bildet dieser also ein abgerundetes Ende, welches auch Biegungen des Führungskatheters gut folgen kann.

Um die Drehkräfte von dem Drehgriff 14 bestmöglich auf den vorgekrümmten Bereich des Führungskatheters 1 übertragen zu können und über die Länge des Drehelementes 8 Torsionen weitgehend auszuschließen, kann dieses Drehelement 8 oder Rohr bevorzugt aus Metall bestehen.

Insgesamt ergibt sich ein Führungskatheter 1, der selbst keine eigene Verstärkung zur Erlangung einer Drehstabilität benötigt, weil er ganz gezielt im Bereich seiner Krümmungen 4 temporär dadurch drehfest gemacht werden kann, daß im Inneren ein Drehelement 8 durch Aufspreizung mit ihm verklemmt und so die Drehkraft genau an die gewünschte Stelle übertragen werden kann.

Der Führungskatheter 1 mit einem inneren Kanal 2 zum Einführen eines Behandlungskatheters oder dergleichen ist in seinem dem distalen Austrittsende 3 benachbarten Bereich vorgebogen oder gekrümmt und kann zu seiner Positionierung beim Einführen oder auch in Gebrauchsstellung verdreht werden. Damit die erforderliche Drehkraft nicht über die gesamte Länge der Wandung 7 des Führungskatheters 1 übertragen werden muß, ist ein im Inneren des Führungskatheters vorschiebbares und an seinem distalen Endbereich lösbar festlegbares Drehelement 8 aus torsionsfestem Werkstoff vorgesehen, welches bevorzugt ein geschlitztes Ende und einen Spreizteil 10 aufweist, womit dieses Drehelement 8 zeitweilig an der Innenseite der Wandung 7 des Führungskatheters 1 verklemmbar ist. Somit kann an einer vorgewählten Stelle die Drehkraft über dieses Drehelement 8 auf den Führungskatheter 1 übertragen werden, so daß er selbst keine Drehsteifigkeit benötigt und entsprechend schlank und geschmeidig ausgebildet sein kann.

## Patentansprüche

1. Führungskatheter (1) mit einem inneren Kanal (2) zum Einführen eines Behandlungskatheters oder dergleichen, wobei der dem distalen Austrittsende (3) benachbarte Bereich des Führungskatheters (1) vorgebogen, gekrümmt oder verformbar und der gesamte Führungskatheter zur Positionierung beim Einführen oder in Gebrauchsstellung drehbar ist, **dadurch gekennzeichnet**, daß er ein in seinem Inneren vorschiebbares und an seinem distalen Endbereich lösbar festlegbares Drehelement (8) aus torsionsfestem Werkstoff aufweist.

2. Führungskatheter nach Anspruch 1, dadurch gekennzeichnet, daß das Drehelement (8) ein flexibler, aber drehfester Hohlkörper, insbesondere ein Rohr oder Schlauch ist, welcher an seinem distalen Ende zum Verklemmen mit der Innenseite des Führungskatheters (1) aufweitbar oder spreizbar ist.

3. Führungskatheter nach Anspruch 1 oder 2, dadurch gekennzeichnet, daß das distale Ende des Drehelementes (8) geschlitzt ist und ein in axialer Richtung innerhalb des Drehelementes (8) verschiebbares Spreizteil (10) zum Aufweiten des geschlitzten Endes gegen eine Rückstellkraft vorgesehen ist.

4. Führungskatheter nach einem der Ansprüche 1 bis 3, dadurch gekennzeichnet, daß als axial bewegliches Spreizteil (10) ein vor dem Spreizen außerhalb der Mündung des Rohres (8) oder Schlauches und seines geschlitzten Bereiches angeordneter, in Richtung gegen das Innere des Rohres oder Schlauches hin sich verjüngender Spreizkörper insbesondere ein Konus, Kegel oder Keil vorgesehen ist, der mit einem Zugelement (11) verbunden ist, und daß das Zugelement (11) durch das Rohr (8) oder dergleichen bis zu dessen proximalem Ende verläuft und an dem proximalen Ende austritt.

5. Führungskatheter nach einem der Ansprüche 1 bis 3, dadurch gekennzeichnet, daß das Spreizteil (10) im Inneren eines sich zur Mündung insbesondere konisch verjüngenden geschlitzten Bereiches des Drehrohres (8) oder dergleichen angeordnet ist und eine mandrinartige Verbindung (11) durch das Rohr bis zu dessen proximalem Ende verläuft und aus diesem mindestens um das Maß vorsteht, um welches das Spreizteil (10) zu der Mündung hin vorschiebbar ist.

6. Führungskatheter nach einem der Ansprüche 1 bis 5, dadurch gekennzeichnet, daß zur Durchführung der relativen Axialbewegung des Spreizkörpers (10) am proximalen Ende des Rohres (8) oder Schlauches ein Verstellgewinde (12) vorgesehen ist, wobei insbesondere das axial verschiebliche Teil ein Gewinde aufweist und das Rohr oder ein damit verbundenes Lagerteil (13) das dazugehörende Gegengewinde hat.

7. Führungskatheter nach einem der Ansprüche 1 bis 6, dadurch gekennzeichnet, daß der Spreizteil (10) zum Aufspreizen und Verklemmen des distalen Endes des Rohres etwa birnenförmig ausgebildet ist, wobei die verdickte Seite das distale Ende des Spreizkörpers (10) bildet und der sich verjüngende Teil zu dem proximalen Ende hin gerichtet ist und das Zugelement (11) zum Einziehen in das geschlitzte Rohrende aufweist.

8. Führungskatheter nach einem der Ansprüche 1 bis 7, dadurch gekennzeichnet, daß das Drehelement (8), insbesondere das Rohr oder der Schlauch, aus Metall besteht.

## Claims

1. A guide catheter (1) having an inner duct (2) for introducing a catheter for medical treatment or a similar device, wherein that region of the guide catheter (1) which is adjacent to the distal exit end (3) is pre-bent, curved or deformable and the entire guide catheter is turnable for positioning when it is introduced or in the position of use, **characterized in that** the guide catheter has a turning element (8) which is made of torsion-resistant material and is adapted to be advanced in the interior of the guide catheter and to be releasably fixed at the distal end zone of the guide catheter.

2. A guide catheter as claimed in claim 1, characterized in that the turning element (8) is a flexible hollow body, but one capable of standing torsional stresses, particularly a tube or hose, which is adapted to be expanded or flared at its distal end so as to be clamped to the inside of the guide catheter (1).

3. A guide catheter as claimed in claim 1 or claim 2, characterized in that the distal end of the turning element (8) is slotted and an expanding means (10) is provided which is movable in the axial direction within the turning element (8) and serves to expand the slotted end against a restoring force.

4. A guide catheter as claimed in any one of claims 1 to 3, characterized in that the axially movable expanding means (10) is provided in the form of an expanding body, particularly a cone or wedge, which, prior to expansion, is arranged outside the opening of the tube (8) or hose and outside the slotted tube area and tapers towards the interior of the tube or hose, said expanding body being connected to a pulling element (11) extending through the tube (8) or the like to depart from said tube at the proximal end thereof.

5. A guide catheter as claimed in any one of claims 1 to 3, characterized in that the expanding means (10) is arranged inside the turning tube (8) or the like, within a slotted zone thereof, particularly within a zone tapering to the opening, and a mandrin-like connection (11) extends through the tube to the proximal end of the tube and projects therefrom at least by the extent to which the expanding means (10) is capable of advancing towards the opening.

6. A guide catheter as claimed in any one of claims 1 to 5, characterized in that an adjustment thread (12) is provided at the proximal end of the tube (8) or hose and serves to perform the relative axial movement of the expanding body (10), whereby in particular the axially movable part has a thread and the tube or a bearing (13) connected thereto has the mating thread.

7. A guide catheter as claimed in any one of claims 1 to 6, characterized in that the expanding means (10) for spreading and clamping the distal end of the tube is generally pear-shaped, whereby the bulged side composes the distal end of the expanding body (10) and the tapering portion points towards the proximal end and has the pulling element (11) for being drawn into the slotted end of the tube.

8. A guide catheter as claimed in any one of claims 1 to 7, characterized in that the turning element (8), particularly the tube or hose, is made of metal.

## Revendications

1. Cathéter de guidage (1) comprenant un canal intérieur (2) destiné à l'introduction d'un cathéter de traitement ou similaire, dans lequel la région du cathéter de guidage (1) qui est voisine de l'extrémité distale de sortie (3) est courbée au préalable, recourbée ou déformable, et la totalité du cathéter de guidage peut être tournée en vue de son positionnement lors de son introduction ou en position d'utilisation, caractérisé par le fait qu'il comporte un élément tournant (8) en un matériau résistant à la torsion, celui-ci pouvant être poussé à l'intérieur du cathéter et fixé d'une manière amovible dans la région de son extrémité distale.

2. Cathéter de guidage selon la revendication 1, caractérisé par le fait que l'élément tournant (8) est un corps creux flexible, mais résistant à la torsion, et en particulier un tube ou un tuyau, qui peut être élargi ou expansé à son extrémité distale en vue de son coincement sur le côté intérieur du cathéter de guidage (1).

3. Cathéter de guidage selon la revendication 1 ou 2, caractérisé par le fait que l'extrémité distale de l'élément tournant (8) est fendue, et qu'en vue de l'élargissement de l'extrémité fendue à l'encontre d'une force de rappel, il est prévu une pièce d'expansion (10) qui peut être déplacée dans la direction axiale à l'intérieur de l'élément tournant (8).

4. Cathéter de guidage selon l'une des revendications 1 à 3, caractérisé par le fait qu'il est prévu, pour servir de pièce d'expansion mobile axialement (10), un corps d'expansion qui est situé avant l'expansion à l'extérieur de l'embouchure du tube (8) ou du tuyau et de sa région fendue, qui se rétrécit dans la direction de l'intérieur du tube ou du tuyau, qui est en particulier un cône, un tronc de cône ou un coin et qui est relié à un élément de traction (11), et par le fait que l'élément de traction (11) s'étend à travers le tube (8) ou similaire jusqu'à l'extrémité proximale de celui-ci, et qu'il sort à l'extrémité proximale.

5. Cathéter de guidage selon l'une des revendications 1 à 3, caractérisé par le fait que la pièce d'expansion (10) est disposée à l'intérieur de la région fendue du tube tournant (8) ou similaire qui se rétrécit en particulier en forme de cône dans la direction de l'embouchure, qu'une liaison (11) analogue à un mandrin s'étend à travers le tube jusqu'à l'extrémité proximale de celui-ci, et qu'elle fait saillie hors de cette extrémité, du moins de la longueur sur laquelle la pièce d'expansion (10) peut être poussée vers l'embouchure.

6. Cathéter de guidage selon l'une des revendications 1 à 5, caractérisé par le fait qu'un filetage de déplacement (12) est prévu à l'extrémité proximale du tube (8) ou du tuyau en vue de réaliser le déplacement axial relatif du corps d'expansion (10), cependant qu'en particulier, la partie qui est mobile axialement présente un filetage, et que le tube ou une pièce de montage (13) qui lui est reliée présente le filetage conjugué associé.

7. Cathéter de guidage selon l'une des revendications 1 à 6, caractérisé par le fait que la pièce d'expansion (10) qui est destinée à l'expansion et au coincement de l'extrémité distale du tube présente à peu près la forme d'une poire, cependant que le côté épaissi constitue l'extrémité distale du corps d'expansion (10), et que la partie qui se rétrécit est dirigée dans la direction de l'extrémité proximale en présentant l'élément de traction (11) qui est destiné à être introduit dans l'extrémité fendue du tube.

8. Cathéter de guidage selon l'une des revendications 1 à 7, caractérisé par le fait que l'élément tournant (8), et en particulier le tube ou le tuyau, est constitué par du métal.
